# EUROPEAN PATENT SPECIFICATION

(11) **EP 4 179 990 B1**
(45) Date of publication and mention of the grant of the patent: **24.12.2025**
(21) Application number: 22206582.3
(22) Date of filing: 10.11.2022
(51) Int. Cl.: A61B 18/14, A61B 18/00, A61B 18/12

(54) **VESSEL SEALER WITH PLASMA BLADE DISSECTION ELECTRODE**
GEFÄSSVERSIEGELUNGSVORRICHTUNG MIT PLASMAKLINGENDISSEKTIONSELEKTRODE
DISPOSITIF DE SCELLEMENT DE VAISSEAUX AVEC ÉLECTRODE DE DISSECTION À LAME DE PLASMA

(30) Priority: 15.11.2021 US 202163279612 P; 24.10.2022 US 202217972158
(43) Date of publication of application: 17.05.2023
(73) Proprietor: Covidien LP, Mansfield, MA 02048 (US)
(72) Inventor: WALBRIDGE, Chelsea E., Boulder, 80301 (US); MANLEY, Prakash, Louisville, 80027 (US); CHENG, Xiaoming, Fort Worth, 76137 (US); MERCIER, Daniel W., 80301, Boulder (US); MILLER, Sean W., Boulder, 80301-3299 (US); BOUCHER, Todd W., Boulder, 80301 (US); KRASTINS, Craig V., Boulder, 80301 (US)
(74) Representative: Maschio & Soames IP Ltd

(56) References cited:
- US-A1- 2021 186 587
- US-A1- 2021 307 812

## Description

### FIELD

The present disclosure relates to surgical instruments and, more particularly, to plasma blades, electrosurgical instruments including plasma blades.

### BACKGROUND

A surgical forceps is a pliers-like instrument that relies on mechanical action between its jaw members to grasp, clamp, and constrict tissue. Electrosurgical forceps utilize both mechanical clamping action and energy to heat tissue to treat, e.g., coagulate, cauterize, or seal, tissue. Prior to tissue treatment, a second device or possibly the same forceps may be utilized to dissect tissue layers or otherwise separate tissue. Once the tissue is separated, the tissue may be treated and, once treated, the surgeon has to accurately sever the treated tissue.

Accordingly, many electrosurgical forceps are designed to include a tip that may be electrically activated to dissect tissue. The forceps may also include a knife that is advanced between the jaw members to cut the treated tissue. As an alternative to a mechanical knife, an energy-based tissue cutting element may be provided to cut the treated tissue using energy, e.g., thermal, electrosurgical, ultrasonic, light, or other suitable energy.
Document US2021/186587A1 describes a thermal cutting element for a surgical instrument. Document US2021/307812A1 describes a method for treating tissue includes determining whether tissue is present between first and second jaw members.

### SUMMARY

The invention is defined by the appended independent claims. Optional features are set out in the appended dependent claims.

As used herein, the term "distal" refers to the portion that is being described which is further from a user, while the term "proximal" refers to the portion that is being described which is closer to a user. Further, to the extent consistent, any or all of the aspects detailed herein may be used in conjunction with any or all of the other aspects detailed herein.

Provided in accordance with aspects of the present disclosure is an end effector for a surgical instrument that includes a pair of opposing first and second jaw members each having a jaw housing supporting an electrically conductive tissue sealing plate disposed thereon. The electrically conductive tissue sealing plates of the first and second jaw members are disposed in opposition relative to one another. One or both of the first or second jaw members is movable relative to the other jaw member to grasp tissue therebetween. The electrically conductive tissue sealing plates of the first and second jaw members are adapted to connect to opposite potentials of an electrosurgical energy source. The electrically conductive tissue sealing plates of the first jaw member has an open T-shaped configuration defining a channel along a length thereof. A plasma blade is disposed within the channel of the electrically conductive tissue sealing plate of the first jaw member and extends to a distal end portion thereof. The plasma blade electrically connects to the energy source and is independently activatable from the electrically conductive tissue sealing plates. The plasma blade includes an insulative material on either side thereof configured to focus electrical and thermal energy to an exposed edge defined along a length of the plasma blade.

In aspects according to the present disclosure, the electrically conductive tissue sealing plate of the second jaw member has an open T-shaped configuration defining a channel along a length thereof and wherein an insulative member is disposed within the channel of the electrically conductive tissue sealing plate of the second jaw member in opposing vertical registration to the plasma blade.

In aspects according to the present disclosure, the insulative member is a made from a compliant high temperature silicone. In other aspects according to the present disclosure, the insulative member is selected from the group consisting of ceramic, parylene, nylon, and PTFE.

In aspects according to the present disclosure, a bridge is disposed within the first jaw member at a proximal end thereof, the bridge configured to provide electrical continuity across the electrically conductive tissue sealing plates of the first and second jaw members.

In aspects according to the present disclosure, a sensor is operably associated with one or both jaw members and is configured to sense when the jaw members are disposed in the open configuration, the sensor communicating with the electrical energy source to configure the electrosurgical instrument for monopolar use upon activation thereof.

In aspects according to the present disclosure, a bipolar activation switch is configured to provide electrical energy to both electrically conductive tissue sealing plates upon activation thereof and a monopolar activation switch configured to provide electrical energy to the plasma blade upon activation thereof. In other aspects according to the present disclosure, a bipolar activation switch is configured to provide electrical energy to both electrically conductive tissue sealing plates upon activation thereof and a monopolar activation switch configured to provide electrical energy to the plasma blade upon activation thereof, wherein the sensor disables power to the bipolar activation switch when the jaw members are disposed in the open configuration.

Provided in accordance with another aspects of the present disclosure is an end effector for a surgical instrument that includes a pair of opposing first and second jaw members each having a jaw housing supporting an electrically conductive tissue sealing plate disposed thereon. The electrically conductive tissue sealing plates of the first and second jaw members are disposed in opposition relative to one another, one or both of the first or second jaw member is movable relative to the other jaw member to grasp tissue therebetween. The electrically conductive tissue sealing plates of the first and second jaw members are adapted to connect to opposite potentials of an electrosurgical energy source. The electrically conductive tissue sealing plates of the first and second jaw member each have an open T-shaped configuration defining a channel along a length thereof.

A plasma blade is disposed within the channel of the electrically conductive tissue sealing plate of the first jaw member and extends to a distal end portion thereof. The plasma blade electrically connects to the energy source and is independently activatable from the electrically conductive tissue sealing plates. An insulative member is disposed within the channel of the electrically conductive tissue sealing plate of the second jaw member in opposing vertical registration to the plasma blade.

In aspects according to the present disclosure, the plasma blade includes an insulative material on either side thereof configured to focus electrical and thermal energy to an exposed edge defined along a length of the plasma blade. In other aspects according to the present disclosure, the insulative member is a made from a compliant high temperature silicone. In still other aspects according to the present disclosure, the insulative member is selected from the group consisting of ceramic, parylene, nylon, and PTFE.

In aspects according to the present disclosure, a bridge disposed within the first jaw member at a proximal end thereof, the bridge configured to provide electrical continuity across the electrically conductive tissue sealing plates of the first and second jaw members.

In aspects according to the present disclosure, a sensor is operably associated with one or both jaw members and is configured to sense when the jaw members are disposed in the open configuration, the sensor communicating with the electrical energy source to configure the electrosurgical instrument for monopolar use upon activation thereof.

In aspects according to the present disclosure, a bipolar activation switch is configured to provide electrical energy to both electrically conductive tissue sealing plates upon activation thereof and a monopolar activation switch configured to provide electrical energy to the plasma blade upon activation thereof. In other aspects according to the present disclosure, a bipolar activation switch is configured to provide electrical energy to both electrically conductive tissue sealing plates upon activation thereof and a monopolar activation switch configured to provide electrical energy to the plasma blade upon activation thereof, wherein the sensor disables power to the bipolar activation switch when the jaw members are disposed in the open configuration.

### BRIEF DESCRIPTION OF DRAWINGS

The above and other aspects and features of the present disclosure will become more apparent in view of the following detailed description when taken in conjunction with the accompanying drawings wherein like reference numerals identify similar or identical elements.
FIG. 1 is a perspective view of a shaft-based electrosurgical forceps provided in accordance with the present disclosure shown connected to an electrosurgical generator;
FIG. 2 is a perspective view of a hemostat-style electrosurgical forceps provided in accordance with the present disclosure;
FIG. 3 is a schematic illustration of a robotic surgical instrument provided in accordance with the present disclosure;
FIG. 4 is a perspective view of a distal end portion of the forceps of FIG. 1, wherein first and second jaw members of an end effector assembly of the forceps are disposed in a spaced-apart position, each jaw member including a respective electrically conductive tissue sealing plate disposed thereon;
FIG. 5A is a bottom, perspective view of the first jaw member of the end effector assembly of FIG. 4;
FIG. 5B is a top, perspective view of the second jaw member of the end effector assembly of FIG. 4;
FIG. 6 is an enlarged, front perspective cross section of respective distal ends the first and second jaw members of FIG. 4 showing a plasma cutting element in accordance with the present disclosure;
FIG. 7 is schematic circuit diagram showing the various electrical connections of the first and second jaw members and the plasma cutting element to a generator for use with the forceps of FIG. 4;
FIGS. 8A and 8B show various views of a bridge associated with the end effector of the surgical instrument configured to provide continuity between electrically conductive tissue sealing plates; and
FIG. 9 shows an enlarged front cross section of the respective distal ends of an additional embodiment of the first and second jaw members of FIG. 4 showing a plasma cutting element in accordance with additional aspects of the present disclosure;

### DETAILED DESCRIPTION

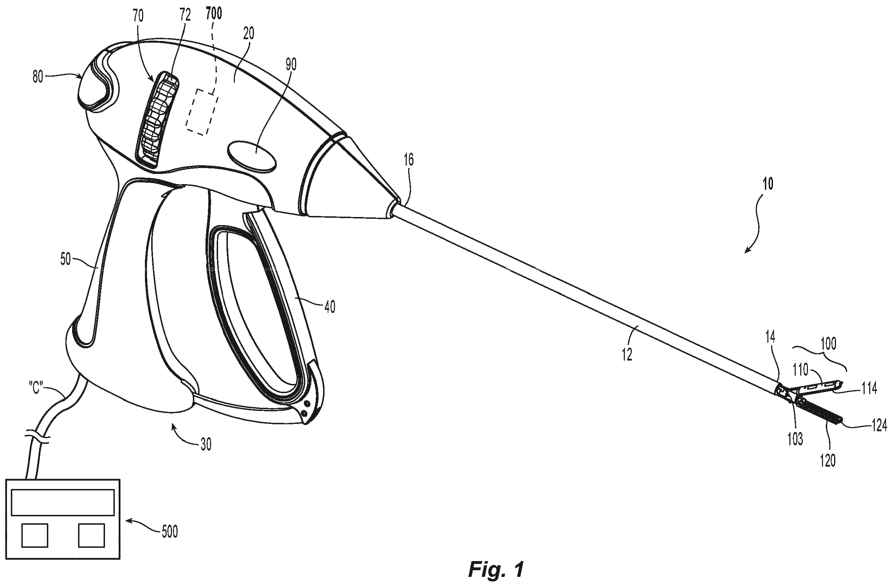

Referring to FIG. 1, a shaft-based electrosurgical forceps provided in accordance with the present disclosure is shown generally identified by reference numeral 10. Aspects and features of forceps 10 not germane to the understanding of the present disclosure are omitted to avoid obscuring the aspects and features of the present disclosure in unnecessary detail.

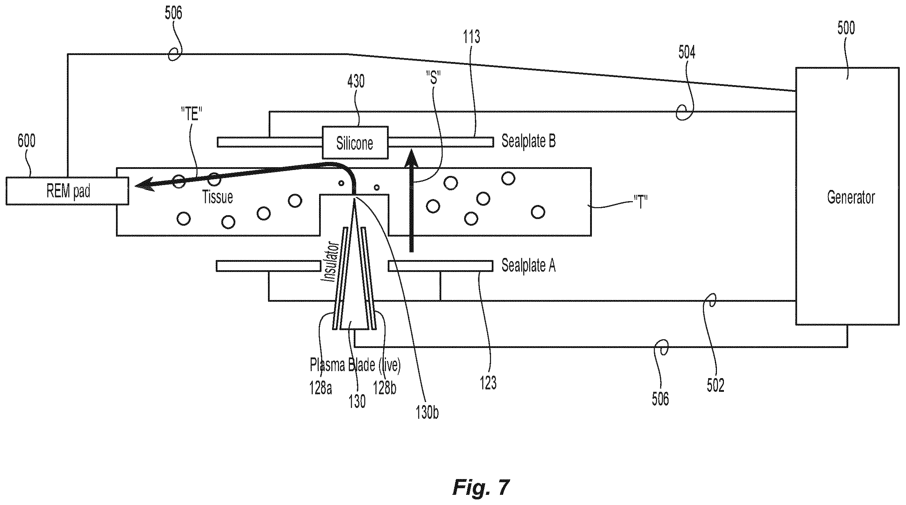

Forceps 10 includes a housing 20, a handle assembly 30, a rotating assembly 70, a first activation switch 80, a second activation switch 90, and an end effector assembly 100. Forceps 10 further includes a shaft 12 having a distal end portion 14 configured to (directly or indirectly) engage end effector assembly 100 and a proximal end portion 16 that (directly or indirectly) engages housing 20. Forceps 10 also includes cable "C" that connects forceps 10 to an energy source, e.g., an electrosurgical generator 500 (FIGS. 1 and 7) Cable "C" includes wires 502, 504 (FIG. 7) extending therethrough that have sufficient length to extend through shaft 12 in order to connect to one or both tissue-treating surfaces 114, 124 of jaw members 110, 120, respectively, of end effector assembly 100 to provide energy thereto. First activation switch 80 is coupled to tissue-treating surfaces 114, 124 and the electrosurgical generator 500 for enabling the selective activation of the supply of energy to jaw members 110, 120 for treating, e.g., cauterizing, coagulating/ desiccating, and/or sealing, tissue. As explained in more detail below, a second activation switch 90 is coupled to a plasma blade 130 of jaw member 120 and the electrosurgical generator 500 via wire 506 for enabling the selective activation of the supply of energy to plasma blade 130 for thermally cutting tissue.

As can be appreciated, after sealing, a vessel or tissue may be separated either by the use of a central cutting electrode that is elevated to a high temperature (e.g., a resistively heated element) or by using an electrosurgical electrode that is polarized opposite of the patient return pad (REM pad) during a so-called "cut" mode or cycle. The electrosurgical electrode used for cutting may either be a traditional uncoated electrode or, according to the present disclosure, employ a plasma blade 130.

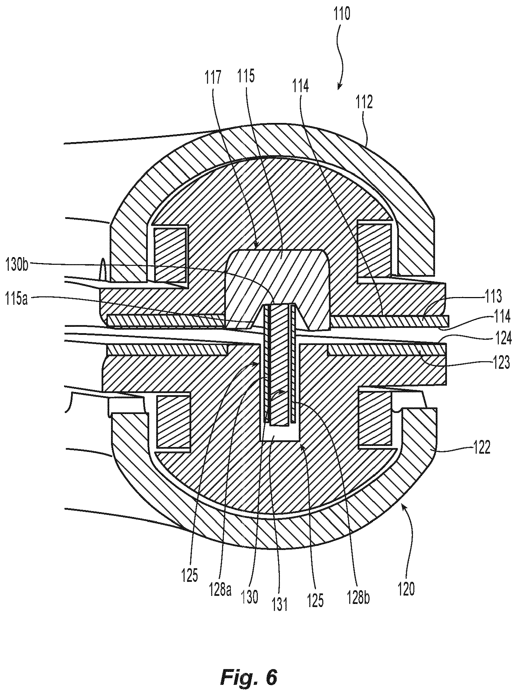

A plasma blade 130 is configured to direct a majority of the electrosurgical energy to an exposed cutting edge, e.g., cutting edge 130b (FIG. 6). Many different configurations for the plasma blade 130 are discussed herein and generally include covering the lateral or side surfaces of the plasma blade 130 with an insulative material. As a result, energy supplied by the generator 500 is forced through a thin strip of material or edge 130b along periphery of the plasma blade 130 unlike an uncoated electrode. It is envisioned that better tissue separation reliability and efficiency may be achieved utilizing the plasma blade 130 as the plasma blade 130 directs energy through the exposed edge 130b on its way to the patient return pad (REM pad) or oppositely polarized sealing plate, e.g., sealing plate 113, during the cut mode or cycle as opposed to the energy taking more favorable alternate paths available laterally that by-pass the tissue. Further, the plasma blade 130 allows the generator 500 to power the cut cycle or cut mode using much lower voltages adding to the overall efficiency of the design.

The cut cycle of a plasma blade 130 also differs from convention electrode cut modes or cut cycles as a result of the plasma blade's 130 particular configuration and the tendency to direct the electrical energy to and out from the cutting edge 130b requiring less energy to effectively cut the tissue. Typically, the energy waveform includes a maximum peak-to-peak voltage of up to about 1000V and a root mean squared voltage (VRms) of up to about 360V. The waveform is typically unpulsed but may be pulsed depending upon a particular purpose.

Handle assembly 30 of forceps 10 includes a fixed handle 50 and a movable handle 40. Fixed handle 50 is integrally associated with housing 20 and handle 40 is movable relative to fixed handle 50. Movable handle 40 of handle assembly 30 is operably coupled to a drive assembly (not shown) that, together, mechanically cooperate to impart movement of one or both of jaw members 110, 120 of end effector assembly 100 about a pivot 103 between a spaced-apart position and an approximated position to grasp tissue between tissue-treating surfaces 114, 124 of jaw members 110, 120. As shown in FIG. 1, movable handle 40 is initially spaced-apart from fixed handle 50 and, correspondingly, jaw members 110, 120 of end effector assembly 100 are disposed in the spaced-apart position. Movable handle 40 is depressible from this initial position to a depressed position corresponding to the approximated position of jaw members 110, 120. Rotating assembly 70 includes a rotation wheel 72 that is selectively rotatable in either direction to correspondingly rotate end effector assembly 100 relative to housing 20.

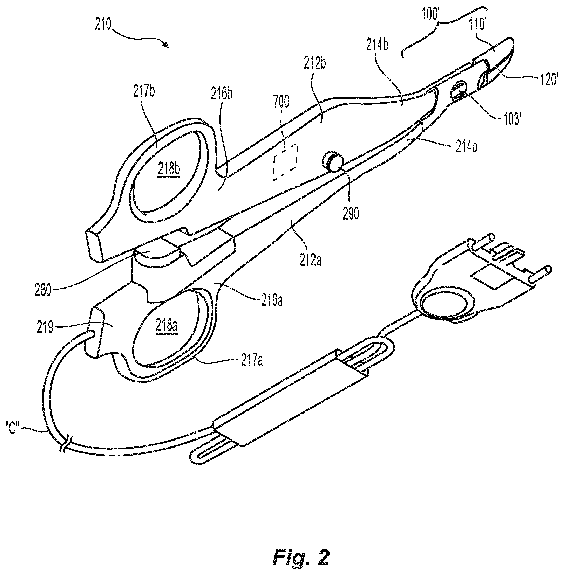

Referring to FIG. 2, a hemostat-style electrosurgical forceps provided in accordance with the present disclosure is shown generally identified by reference numeral 210. Aspects and features of forceps 210 not germane to the understanding of the present disclosure are omitted to avoid obscuring the aspects and features of the present disclosure in unnecessary detail.

Forceps 210 includes two elongated shaft members 212a, 212b, each having a proximal end portion 216a, 216b, and a distal end portion 214a, 214b, respectively. Forceps 210 is configured for use with an end effector assembly 100' similar to end effector assembly 100. More specifically, end effector assembly 100' includes first and second jaw members 110', 120' attached to respective distal end portions 214a, 214b of shaft members 212a, 212b. Jaw members 110', 120' are pivotably connected about a pivot 103'. Each shaft member 212a, 212b includes a handle 217a, 217b disposed at the proximal end portion 216a, 216b thereof. Each handle 217a, 217b defines a finger hole 218a, 218b therethrough for receiving a finger of the user. As can be appreciated, finger holes 218a, 218b facilitate movement of the shaft members 212a, 212b relative to one another to, in turn, pivot jaw members 110', 120' from the spaced-apart position, wherein jaw members 110', 120' are disposed in spaced relation relative to one another, to the approximated position, wherein jaw members 110', 120' cooperate to grasp tissue therebetween.

One of the shaft members 212a, 212b of forceps 210, e.g., shaft member 212b, includes a proximal shaft connector 219 configured to connect forceps 210 to a source of energy, e.g., electrosurgical generator 500 (FIGS.1 and 7). Proximal shaft connector 219 secures a cable "C" to forceps 210 such that the user may selectively supply energy to jaw members 110', 120' for treating tissue. More specifically, a first activation switch 280 is provided for supplying energy to jaw members 110', 120' to treat tissue upon sufficient approximation of shaft members 212a, 212b, e.g., upon activation of first activation switch 280 via shaft member 212a. A second activation switch 290 disposed on either or both of shaft members 212a, 212b is coupled to the plasma blade (e.g., similar to plasma blade 130 of jaw member 120) operably associated with one of the jaw members 110', 120' of end effector assembly 100' and to the electrosurgical generator 500 for enabling the selective activation of the supply of energy to the plasma blade 130 for thermally cutting tissue.

Jaw members 110', 120' define a curved configuration wherein each jaw member is similarly curved laterally off of a longitudinal axis of end effector assembly 100'. However, other suitable curved configurations including curvature towards one of the jaw members 110, 120' (and thus away from the other), multiple curves with the same plane, and/or multiple curves within different planes are also contemplated. Jaw members 110, 120 of end effector assembly 100 (FIG. 1) may likewise be curved according to any of the configurations noted above or in any other suitable manner.

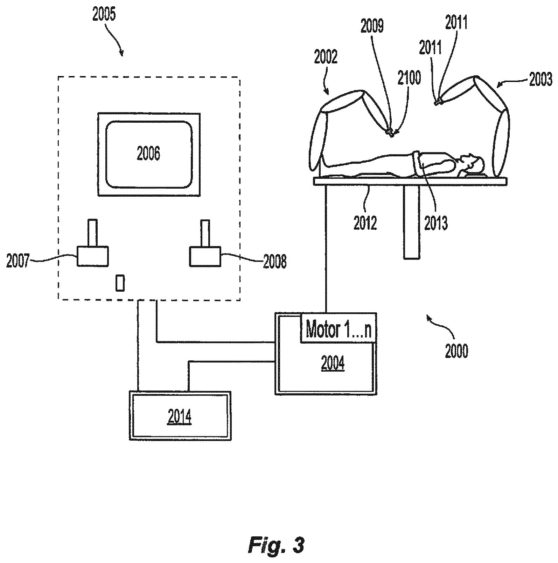

Referring to FIG. 3, a robotic surgical instrument provided in accordance with the present disclosure is shown generally identified by reference numeral 2000. Aspects and features of robotic surgical instrument 2000 not germane to the understanding of the present disclosure are omitted to avoid obscuring the aspects and features of the present disclosure in unnecessary detail.

Robotic surgical instrument 2000 includes a plurality of robot arms 2002, 2003; a control device 2004; and an operating console 2005 coupled with control device 2004. Operating console 2005 may include a display device 2006, which may be set up in particular to display three-dimensional images; and manual input devices 2007, 2008, by means of which a surgeon may be able to telemanipulate robot arms 2002, 2003 in a first operating mode. Robotic surgical instrument 2000 may be configured for use on a patient 2013 lying on a patient table 2012 to be treated in a minimally invasive manner. Robotic surgical instrument 2000 may further include a database 21014, in particular coupled to control device 2004, in which are stored, for example, pre-operative data from patient 2013 and/or anatomical atlases.

Each of the robot arms 2002, 2003 may include a plurality of members, which are connected through joints, and an attaching device 2009, 2011, to which may be attached, for example, an end effector assembly 2100, 2200, respectively. End effector assembly 2100 is similar to end effector assembly 100, although other suitable end effector assemblies for coupling to attaching device 2009 are also contemplated. End effector assembly 2200 may be any end effector assembly, e.g., an endoscopic camera, other surgical tool, etc. Robot arms 2002, 2003 and end effector assemblies 2100, 2200 may be driven by electric drives, e.g., motors, that are connected to control device 2004. Control device 2004 (e.g., a computer) may be configured to activate the motors, in particular by means of a computer program, in such a way that robot arms 2002, 2003, their attaching devices 2009, 2011, and end effector assemblies 2100, 2200 execute a desired movement and/or function according to a corresponding input from manual input devices 2007, 2008, respectively. Control device 2004 may also be configured in such a way that it regulates the movement of robot arms 2002, 2003 and/or of the motors.

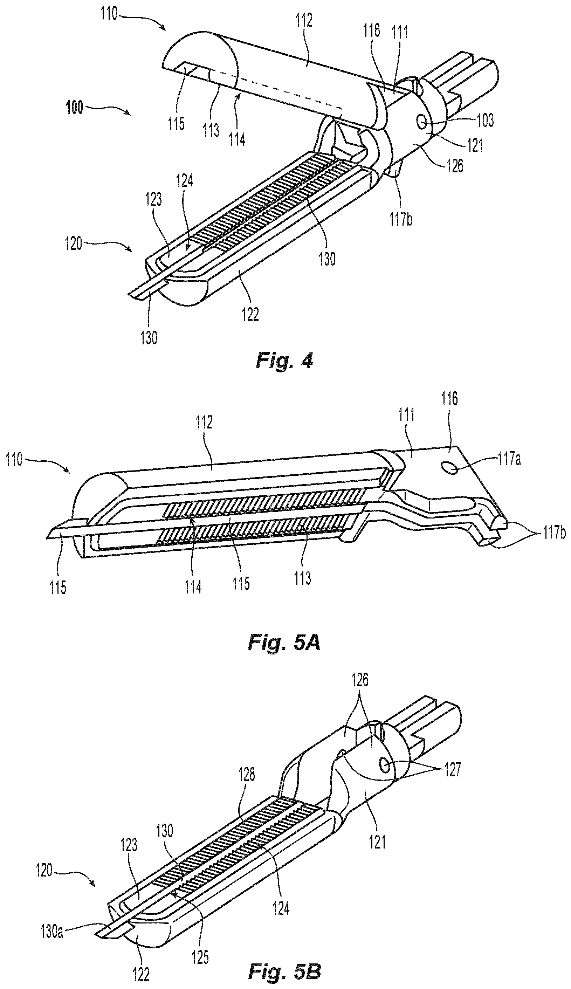

Turning to FIG. 4, one embodiment of a known end effector assembly 100, as noted above, includes first and second jaw members 110, 120. Each jaw member 110, 120 may include a structural frame 111, 121, a jaw housing 112, 122, and a tissue-treating plate 113, 123 defining the respective tissue-treating surface 114, 124 thereof. Alternatively, only one of the jaw members, e.g., jaw member 120, may include the structural frame 121, jaw housing 122, and tissue-treating plate 123 defining the tissue-treating surface 124. In such embodiments, the other jaw member, e.g., jaw member 110, may be formed as a single unitary body, e.g., a piece of conductive material acting as the structural frame 111 and jaw housing 112 and defining the tissue-treating surface 114. An outer surface of the jaw housing 112, in such embodiments, may be at least partially coated with an insulative material or may remain exposed. For the purposes herein, the term "insulative" is defined as thermal or electrical conductivity that is lower than the adjacent materials of the jaw members 110, 120. Materials or coatings described herein may be thermally insulative, electrically insulative or both.

In embodiments, tissue-treating plates 113, 123 may be deposited onto jaw housings 112, 122 or jaw inserts (not shown) disposed within jaw housings 112, 122, e.g., via sputtering. Alternatively, tissue-treating plates 113, 123 may be pre-formed and engaged with jaw housings 112, 122 and/or jaw inserts (not shown) disposed within jaw housings 112, 122 via, for example, overmolding, adhesion, mechanical engagement, etc.

Referring in particular to FIGS. 4-5B, jaw member 110, as noted above, may be configured similarly as jaw member 120, may be formed as a single unitary body, or may be formed in any other suitable manner so as to define a structural frame 111 and a tissue-treating surface 114 opposing tissue-treating surface 124 of jaw member 120. Structural frame 111 includes a proximal flange portion 116 about which jaw member 110 is pivotably coupled to jaw member 120. In shaft-based or robotic embodiments, proximal flange portion 116 may further include an aperture 117a for receipt of pivot 103 and at least one protrusion 117b extending therefrom that is configured for receipt within an aperture defined within a drive sleeve of the drive assembly (not shown) such that translation of the drive sleeve, e.g., in response to actuation of movable handle 40 (FIG. 1) or a robotic drive, pivots jaw member 110 about pivot 103 and relative to jaw member 120 between the spaced-apart position and the approximated position. However, other suitable drive arrangements are also contemplated, e.g., using cam pins and cam slots, a screw-drive mechanism, etc.

Regardless of the particular configuration of jaw member 110, jaw member 110 may include a longitudinally-extending insulative member 115 extending along at least a portion of the length of tissue-treating surface 114. Insulative member 115 may be transversely centered on tissue-treating surface 114 or may be offset relative thereto. Further, insulative member 115 may be disposed, e.g., deposited, coated, etc., on tissue-treating surface 114, may be positioned within a channel or recess defined within tissue-treating surface 114, or may define any other suitable configuration. Additionally, insulative member 115 may be substantially (within manufacturing, material, and/or use tolerances) coplanar with tissue-treating surface 114, may protrude from tissue-treating surface 114, may be recessed relative to tissue-treating surface 114, or may include different portions that are coplanar, protruding, and/or recessed relative to tissue-treating surface 114. Insulative member 115 may be formed from, for example, compliant high temperature silicone, ceramic, parylene, nylon, PTFE, or other suitable material(s) (including combinations of insulative and non-insulative materials). The insulative member 115 may also be formed from polybenzimidazole or similar materials.

With reference to FIGS. 4 and 5B, as noted above, jaw member 120 includes a structural frame 121, a jaw housing 122, and a tissue-treating plate 123 defining the tissue-treating surface 124 thereof. Jaw member 120 further includes a plasma blade 130. Structural frame 121 defines a proximal flange portion 126 and a distal body portion (not shown) extending distally from proximal flange portion 126. Proximal flange portion 126 is bifurcated to define a pair of spaced-apart proximal flange portion segments that receive proximal flange 111 of jaw member 110 therebetween and define aligned apertures 127 configured for receipt of pivot 103 therethrough to pivotably couple jaw members 110, 120 with one another (FIG. 4).

Jaw housing 122 of jaw member 120 is disposed about the distal body portion of structural frame 121, e.g., via overmolding, adhesion, mechanical engagement, etc., and supports tissue-treating plate 123 thereon, e.g., via overmolding, adhesion, mechanical engagement, depositing (such as, for example, via sputtering), etc.

As shown in FIG. 6, tissue sealing plate 123 is generally open T-shaped to define a longitudinally-extending slot 125 therealong for housing the plasma blade 130 therein. Tissue-treating plate 123, as noted above, defines tissue-treating surface 124. Longitudinally-extending slot 125 is defined through tissue-treating plate 123 and is positioned to oppose insulative member 115 of jaw member 110 (FIG. 5A) in the approximated position. Slot 125 may extend through a portion of jaw housing 122, a jaw insert (if so provided), and/or other components of jaw member 120 to enable receipt of the plasma blade 130 at least partially within slot 125.

Plasma blade 130 is partially covered on either side with an insulative material 128a, 128b along a length thereof to direct the energy (electrical and thermal) to exposed surfaces, e.g., a top surface 130b of the plasma blade 130. In embodiments, insulative material 128a, 128b (glass, ceramic, etc.) covers the entire surface of the plasma blade 130 except the exposed top surface or cutting edge 130b. Plasma blade 130 may be configured to contact insulative member 115 (FIG. 5A) in the approximated position and may be configured to regulate (or contribute to the regulation of) a gap distance between tissue-treating surfaces 114, 124 in the approximated position. Alternatively or additionally, one or more stop members (not shown) associated with jaw member 110 and/or jaw member 120 may be provided to regulate the gap distance between tissue-treating surfaces 114, 124 in the approximated position.

As mentioned above, plasma blade 130 is surrounded by an insulative material 128a, 128b disposed within slot 125 or attached to the plasma blade 130 to both electrically the isolate plasma blade 130 from tissue-treating plate 123 and to direct energy to the exposed edge or top surface 130b for cutting tissue. Plasma blade 130 and insulative material 128a, 128b may similarly or differently be substantially (within manufacturing, material, and/or use tolerances) coplanar with tissue-treating surface 124, may protrude from tissue-treating surface 124, may be recessed relative to tissue-treating surface 124, or may include different portions that are coplanar, protruding, and/or recessed relative to tissue-treating surface 124. The insulative materials 128a, 128b may be directed deposited onto the plasma blade 130, may be taped onto the plasma blade 130, may be configured to encapsulated or cover the plasma blade 130, may form a pocket for receiving the plasma blade 130, may be molded to the plasma blade 130 or in any other fashion known in the art.

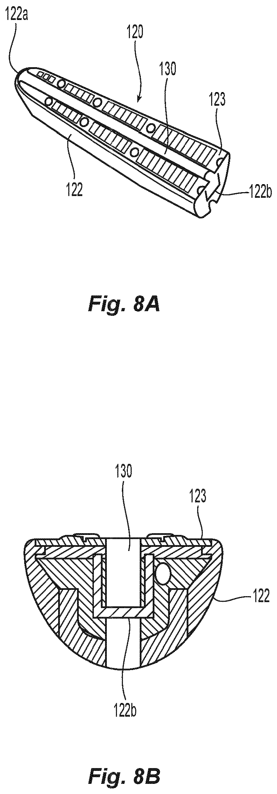

As can be appreciated, configuring seal plate 123 in an open T-shape simplifies electrical connections as only a single point of electrical connection is required with the integral, yet un-bisected design. The open T-shaped configuration allows the plasma blade 130 to extend beyond the distal end of the jaw member 120 for dissection purposes, if desired. A bridge 122b may be disposed at the proximal end of the jaw member 120 to provide electrical continuity across the seal plate 123 and simplify manufacturing, electrical connection and assembly. The bridge 122b allows the seal plate 123 to remain split along the entire length of the jaw member 120 (FIGS. 8A and 8B) and retain electrical continuity across the same.

Jaw member 110 also includes seal plate 113 affixed thereto in opposing relation to jaw member 120. Similarly, seal plate 113 is generally open T-shaped along a length thereof and includes a channel 117 defined therealong that is configured to house insulative member 115 therein. Insulative member 115 is disposed in vertical registration with plasma blade 130.

FIG. 7 illustrates the various electrical connections to the sealing plate 113, 123 and the plasma blade 130 to the generator 500 to allow both bipolar sealing and monopolar dissection. More particularly, seal plates 113, 123 are electrically connected to the generator via cable "C" (FIG. 1) that houses wires, 504, 502, respectively. As mentioned above, the open T-shaped configuration of both plates 113, 123 simplifies assembly requiring only one electrical connection to each plate. Plasma blade 130 is also connected to the generator 500 via wire 506 disposed within cable "C". A return electrode monitoring (REM) connection 600 (e.g., return pad, plate, etc.) may be connected to the patient or proximate the tissue site to provide and alternative return path for electrical energy during monopolar activation. Alternatively, seal plate 113 may be configured to act as an electrical return.

During bipolar sealing, the generator 500 provides electrical energy to seal plates 113, 123 to seal tissue "T" disposed therebetween according to one or more known sealing algorithms. As a result thereof, during activation, a tissue seal "S" is created between sealing plates 113, 123 on either side of channels 117 and 125 respectively. Once sealed, the algorithm may be configured to automatically activate the plasma blade 130 to transect the tissue "T" substantially along the center of the opposing seal plates 113, 123. As mentioned above, the upper surface or edge 130b of the plasmas blade 130 is the only portion thereof that is exposed thereby eliminating any arc effect (to other conductors) and focusing the electrical and thermal energy along a defined cutting path. During activation of the plasma blade 130 and one or more of the sealing plates 113, 123 may remain electrically energized, e.g., sealing plate 113, to act as an electrical return to generator 500. Alternatively, a return pad 600 (e.g., REM system) may be electrically energized and act as the electrical return for the plasma blade 130.

As mentioned above, the plasma blade 130 may be utilized for monopolar dissection, e.g., open jaw dissection. More particularly, with the jaw members 110, 120 disposed in an open configuration, the plasma blade 130 may be activated via switch 90 with a first electrical potential which is directed through the tissue and to a return pad 600 (e.g., REM system) having a second electrical potential. Tissue may be dissected as jaw member 120 is moved into contact therewith.

FIGS. 8A and 8B show the open T-shaped configuration of seal plate 123 wherein, unlike conventional seal plates, the plasma blade 130 extends to the distal tip 122a of the jaw member 120 between the open-ended seal plate 123. The plasma blade 130 may also be configured to extend to a point proximate the distal tip 122a or beyond the distal tip 122a. The bridge 122b which may be buried in the housing 122 connects across the proximal end of the seal plate 123 to retain electrical continuity.

Generally referring to FIGS. 1-5B, tissue-treating plates 113, 123 are formed from an electrically conductive material, e.g., for conducting electrical energy therebetween for treating tissue, although tissue-treating plates 113, 123 may alternatively be configured to conduct any suitable energy, e.g., thermal, microwave, light, ultrasonic, etc., through tissue grasped therebetween for energy-based tissue treatment. As mentioned above, tissue-treating plates 113, 123 are coupled to activation switch 80 and electrosurgical generator "G" (FIG. 1) such that energy may be selectively supplied to tissue-treating plates 113, 123 and conducted therebetween and through tissue disposed between jaw members 110, 120 to treat tissue, e.g., seal tissue on either side and extending across plasma blade 130.

Plasma blade 130, on the other hand, is configured to connect to electrosurgical generator 500 (FIG. 1) and second activation switch 90 to enable selective activation of the supply of energy to plasma blade 130 for heating the tip 130b of plasma blade 130 to thermally cut tissue disposed between jaw members 110, 120, e.g., to cut the sealed tissue into first and second sealed tissue portions. Insulative materials 128a, 128b are disposed on either side of the plasma blade 130 to focus the thermal energy to the tip 130b. The plasma blade 130 may be controlled by the surgeon, may be automatically controlled by one or more parameters or inputs or feedback associated with the generator 500 or by an algorithm. One or multiple switches 80, 90 may be utilized to accomplish this purpose. For example, if a single switch is utilized, the generator 500 may stop activation of the tissue-treating plates 113, 123 once the seal cycle is complete and automatically initiate activation of the plasma blade 130. Sealing and transection of tissue may be simultaneous or sequential depending upon the particular algorithm being utilized. Other configurations including multi-mode switches, other separate switches, etc. may alternatively be provided. Cross reference is made to U.S. Provisional Patent Application Serial Nc 69/952.232.

A sensor 700 (FIGS 1 and 2) may be disposed within the housing 20, handle 50 or generator 500 that senses when the jaw members 110, 120 are disposed in the open configuration and automatically configures the forceps 10 (or forceps 210) for open monopolar dissection. The sensor 700 may be designed to automatically configure the sealing plates 113, 123, plasma blade 130 and possible a return pad 600 for monopolar use upon sensing the jaw members 110, 120 are disposed in an open configuration. Moreover, the sensor 700 may cut power to the bipolar switch 80 and allow only switch 90 to be operable upon sensing the jaw members 110, 120 are disposed in an open configuration or, with a single switch system, automatically configure the instrument 10, 210 for bipolar sealing or monopolar dissection depending on the position of the jaw members 110, 120.

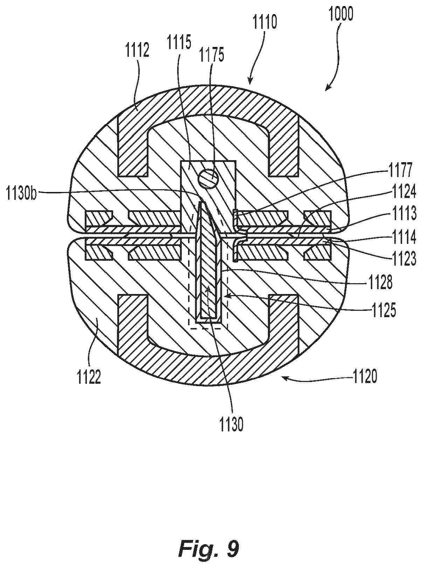

FIG. 9 shows an end effector 1000 that is generally similar to end effector 100 and as such will only be described in sufficient detail to note the differences therebetween. End effector assembly 1000 includes first and second jaw members 1110, 1120 each including respective jaw housings 112, 122 and tissue-treating plates 1113, 1123 defining the respective tissue-treating surface 1114, 1124 thereof.

Tissue sealing plate 1123 is generally open T-shaped to define a longitudinally-extending slot 1125 therealong for housing the plasma blade 1130 therein. Longitudinally-extending slot 1125 is defined through tissue-treating plate 1123 and is positioned to oppose insulative member 1115 of jaw member 1110 in the approximated position. Slot 1125 may extend through a portion of jaw housing 1122 and/or other components of jaw member 1120 to enable receipt of the plasma blade 1130 at least partially within slot 1125.

Plasma blade 1130 is partially covered on either side with an insulative material 1128 along a length thereof to direct the energy (electrical and thermal) to exposed surfaces, e.g., a top surface 1130b of the plasma blade 1130. In embodiments or methods, the plasma blade 1130 may be encapsulated in glass via a so-called dip and cure method or alternatively the glass may be screen printed thereon. In other embodiments or methods, a ceramic may be sprayed onto the sides of the plasma blade 1130 leaving the edge 1130b exposed. The ceramic may be deposited onto the plasma blade 1130 via electrolytic oxidation.

In yet other embodiments or methods, a polyamide such as the polyamide sold under the trademark Kapton^{®} may be taped or film coated onto the sides of the plasma blade 1130 leaving edge 1130b exposed. In other embodiments or methods, molded engineering plastics may be disposed on either side of the plasma blade 1130, e.g., plastics such as polyphthalamide (PPA) such as that sold under the trademark Amodel^{®}, polyetheretherketone (PEEK), polybenzimidazole (PBI), etc. A synthetic fluoropolymer such as polytetrafluoroethylene (PTFE) may also be disposed on either side of the plasma blade 1130. In still other embodiments, silicone may be molded on either side of the plasma blade 1130. In some of the above applications or methods, post grinding may be necessary reveal the exposed edge 1130b. In other instances, one or more techniques may be utilized to automatically expose the edge 1130b during the process of insulating the sides of the plasma blade 1130.

Plasma blade 1130 may be configured to contact insulative member 1115 in the approximated position and may be configured to regulate (or contribute to the regulation of) a gap distance between tissue-treating surfaces 1114, 1124 in the approximated position. In addition to using a return pad or REM pad (not shown), the insulative member 1115 may be configured to direct electrical energy away from the sealed tissue. For example, a return electrode 1175 may be embedded into the insulative member 1115 and connected to the electrical return. As such, the embedded electrode 1175 directs electrical energy from the plasma blade 1130 away from the seal plates and tissue. Other configurations and methods of directing electrical energy may also be employed, e.g., utilizing a monolithic opposing jaw, e.g., jaw member 1110, to act as the electrical return or the opposing seal plate, e.g., seal plate 1113.

In other aspects according to the present disclosure, a coating 1177 may be applied to the inner peripheral edges of the seal plates 1113, 1123 to impede electrical energy towards the tissue. For example, a high temperature silicone such as SiO2 may be employed for this purpose.

Referring back to FIGS. 1-6 and as mentioned above, plasma blade 130 is surrounded by an insulative material 128a, 128b disposed within slot 125 or attached to the plasma blade 130 to both electrically the isolate plasma blade 130 from tissue-treating plate 123 and to direct energy to the exposed edge or top surface 130b for cutting tissue. Plasma blade 130 and insulative material 128a, 128b may similarly or differently be substantially (within manufacturing, material, and/or use tolerances) coplanar with tissue-treating surface 124, may protrude from tissue-treating surface 124, may be recessed relative to tissue-treating surface 124, or may include different portions that are coplanar, protruding, and/or recessed relative to tissue-treating surface 124. The insulative materials 128a, 128b may be directed deposited onto the plasma blade 130, may be taped onto the plasma blade 130, may be configured to encapsulated or cover the plasma blade 130, may form a pocket for receiving the plasma blade 130, may be molded to the plasma blade 130 or in any other fashion known in the art.

While several embodiments of the disclosure have been shown in the drawings, it is not intended that the disclosure be limited thereto, as it is intended that the disclosure be as broad in scope as the art will allow and that the specification be read likewise. For example, the plasma blade 130 may be segmented along the length of the jaw members 110, 120 and independently activatable depending upon a particular purpose. Therefore, the above description should not be construed as limiting, but merely as exemplifications of particular embodiments. Those skilled in the art will envision other modifications within the scope of the claims appended hereto.

## Claims

1. An end effector assembly (100, 1000) for an electrosurgical instrument, comprising:
a pair of opposing first and second jaw members (110, 120, 1110, 1120) each including a jaw housing supporting an electrically conductive tissue sealing plate (113, 123, 1113, 1123) disposed thereon, the electrically conductive tissue sealing plates of the first and second jaw members disposed in opposition relative to one another, at least one of the first or second jaw member movable relative to the other jaw member to grasp tissue therebetween, the electrically conductive tissue sealing plates of the first and second jaw members adapted to connect to opposite potentials of an electrosurgical energy source, the electrically conductive tissue sealing plate of the first jaw member having an open T-shaped configuration defining a channel (125, 1125) along a length thereof; and
a plasma blade (130, 1130) disposed within the channel (125, 1125) of the electrically conductive tissue sealing plate of the first jaw member and extending to a distal end portion thereof, the plasma blade electrically connected to the energy source and independently activatable from the electrically conductive tissue sealing plates, the plasma blade having opposing sides and a top cutting edge (130b, 1130b), the blade including an insulative material (128, 1128) covering the surface of the blade except the top cutting edge to provide an exposed edge to focus electrical and thermal energy to the exposed edge (130b, 1130b) defined along a length of the plasma blade.

2. The end effector assembly according to claim 1, wherein the electrically conductive tissue sealing plate of the second jaw member has an open T-shaped configuration defining a channel along a length thereof and wherein an insulative member (115, 1115) is disposed within the channel of the electrically conductive tissue sealing plate of the second jaw member in opposing vertical registration to the plasma blade.

3. The end effector assembly according to claim 2, wherein the insulative member (115, 1115) is a made from a compliant high temperature silicone.

4. The end effector assembly according to claim 2 or claim 3, wherein the insulative member (115, 1115) is selected from the group consisting of ceramic, parylene, nylon, and PTFE.

5. The end effector assembly according to any preceding claim, further comprising a bridge (122b) disposed within the first jaw member at a proximal end thereof, the bridge configured to provide electrical continuity across the electrically conductive tissue sealing plates of the first and second jaw members.

6. The end effector assembly according to any preceding claim, further comprising a sensor (700) operably associated with at least one of the jaw members and configured to sense when the jaw members are disposed in the open configuration, the sensor communicating with the electrical energy source to configure the electrosurgical instrument for monopolar use upon activation thereof.

7. The end effector assembly according to any preceding claim, further comprising a bipolar activation switch (80) configured to provide electrical energy to both electrically conductive tissue sealing plates upon activation thereof and a monopolar activation switch (90) configured to provide electrical energy to the plasma blade (130, 1130) upon activation thereof.

8. The end effector assembly according to claim 6, further comprising a bipolar activation switch (80) configured to provide electrical energy to both electrically conductive tissue sealing plates upon activation thereof and a monopolar activation switch (90) configured to provide electrical energy to the plasma blade upon activation thereof, wherein the sensor (700) disables power to the bipolar activation switch when the jaw members are disposed in the open configuration.

## Patentansprüche

1. Endeffektorbaugruppe (100, 1000) für ein elektrochirurgisches Instrument, umfassend:
ein Paar von gegenüberliegenden ersten und zweiten Backenelementen (110, 120, 1110, 1120), die jeweils ein Backengehäuse enthalten, das eine darauf angeordnete elektrisch leitfähige Gewebeversiegelungsplatte (113, 123, 1113, 1123) trägt, wobei die elektrisch leitfähigen Gewebeversiegelungsplatten des ersten und zweiten Backenelements einander gegenüberliegend angeordnet sind, wobei wenigstens eines von dem ersten und dem zweiten Backenelement relativ zu dem anderen Backenelement beweglich ist, um Gewebe dazwischen zu greifen, wobei die elektrisch leitfähigen Gewebeversiegelungsplatten des ersten und des zweiten Backenelements dafür ausgelegt sind, mit entgegengesetzten Potentialen einer elektrochirurgischen Energiequelle verbunden zu werden, wobei die elektrisch leitfähige Gewebeversiegelungsplatte des ersten Backenelements eine offene T-förmige Konfiguration aufweist, die einen Kanal (125, 1125) entlang einer Länge davon definiert; und
eine Plasmaklinge (130, 1130), die in dem Kanal (125, 1125) der elektrisch leitfähigen Gewebeversiegelungsplatte des ersten Backenelements angeordnet ist und sich zu einem distalen Endabschnitt davon erstreckt, wobei die Plasmaklinge elektrisch mit der Energiequelle verbunden ist und unabhängig von den elektrisch leitfähigen Gewebeversiegelungsplatten aktivierbar ist, wobei die Plasmaklinge gegenüberliegende Seiten und eine obere Schneidkante (130b, 1130b) aufweist, wobei die Klinge ein isolierendes Material (128, 1128) umfasst, das die Oberfläche der Klinge mit Ausnahme der oberen Schneidkante bedeckt, um eine freiliegende Kante bereitzustellen, um elektrische und thermische Energie auf die freiliegende Kante (130b, 1130b) zu fokussieren, die entlang einer Länge der Plasmaklinge definiert ist.

2. Endeffektorbaugruppe nach Anspruch 1, wobei die elektrisch leitfähige Gewebeversiegelungsplatte des zweiten Backenelements eine offene T-förmige Konfiguration aufweist, die einen Kanal entlang einer Länge davon definiert, und wobei ein isolierendes Element (115, 1115) in dem Kanal der elektrisch leitfähigen Gewebeversiegelungsplatte des zweiten Backenelements in gegenüberliegender vertikaler Ausrichtung zu der Plasmasklinge angeordnet ist.

3. Endeffektorbaugruppe nach Anspruch 2, wobei das isolierende Element (115, 1115) aus einem nachgiebigen Hochtemperatursilikon hergestellt ist.

4. Endeffektorbaugruppe nach Anspruch 2 oder Anspruch 3, wobei das isolierende Element (115, 1115) ausgewählt ist aus der Gruppe bestehend aus Keramik, Parylen, Nylon und PTFE.

5. Endeffektorbaugruppe nach einem der vorstehenden Ansprüche, ferner umfassend eine Brücke (122b), die innerhalb des ersten Backenelements an einem proximalen Ende davon angeordnet ist, wobei die Brücke dafür gestaltet ist, elektrische Kontinuität über die elektrisch leitfähigen Gewebeversiegelungsplatten des ersten und des zweiten Backenelements bereitzustellen.

6. Endeffektorbaugruppe nach einem der vorstehenden Ansprüche, ferner umfassend einen Sensor (700), der betriebsfähig mit wenigstens einem der Backenelemente verbunden ist und dafür gestaltet ist, zu erfassen, wann die Backenelemente in der offenen Konfiguration angeordnet sind, wobei der Sensor mit der elektrischen Energiequelle kommuniziert, um bei Aktivierung davon das elektrochirurgische Instrument zur monopolaren Verwendung zu gestalten.

7. Endeffektorbaugruppe nach einem der vorstehenden Ansprüche, ferner umfassend einen bipolaren Aktivierungsschalter (80), der dafür gestaltet ist, bei Aktivierung davon elektrische Energie an beide elektrisch leitfähigen Gewebeversiegelungsplatten bereitzustellen, und einen monopolaren Aktivierungsschalter (90), der dafür gestaltet ist, bei Aktivierung davon elektrische Energie an die Plasmasklinge (130, 1130) bereitzustellen.

8. Endeffektorbaugruppe nach Anspruch 6, ferner umfassend einen bipolaren Aktivierungsschalter (80), der dafür gestaltet ist, bei Aktivierung davon elektrische Energie an beide leitfähigen Gewebeversiegelungsplatten bereitzustellen, und einen monopolaren Aktivierungsschalter (90), der dafür gestaltet ist, bei Aktivierung davon elektrische Energie an die Plasmaklinge bereitzustellen, wobei der Sensor (700) Leistung an den bipolaren Aktivierungsschalter deaktiviert, wenn die Backenelemente in der offenen Konfiguration angeordnet sind.

## Revendications

1. Ensemble effecteur terminal (100, 1000) pour un instrument électrochirurgical, comprenant :
une paire de premier et second éléments de mâchoire opposés (110, 120, 1110, 1120) comprenant chacun un logement de mâchoire supportant une plaque de scellement tissulaire électriquement conductrice (113, 123, 1113, 1123) disposée sur celui-ci, les plaques de scellement tissulaire électriquement conductrices des premier et second éléments de mâchoire étant disposées en opposition l'une par rapport à l'autre, au moins l'un des deux éléments de mâchoire étant mobile par rapport à l'autre pour saisir le tissu entre eux, les plaques de scellement tissulaire électriquement conductrices des premier et second éléments de mâchoire étant conçues pour être connectées aux potentiels opposés d'une source d'énergie électrochirurgicale, la plaque de scellement tissulaire électriquement conductrice du premier élément de mâchoire présentant une configuration en forme de T ouvert définissant un canal (125, 1125) sur une longueur de celle-ci ; et
une lame de plasma (130, 1130) disposée dans le canal (125, 1125) de la plaque de scellement tissulaire électriquement conductrice du premier élément de mâchoire et s'étendant jusqu'à une partie d'extrémité distale de celle-ci, la lame de plasma étant électriquement connectée à la source d'énergie et pouvant être activée indépendamment des plaques de scellement tissulaire électriquement conductrices, la lame de plasma présentant des côtés opposés et un bord tranchant supérieur (130b, 1130b), la lame comprenant un matériau isolant (128, 1128) recouvrant la surface de la lame à l'exception du bord tranchant supérieur pour fournir un bord exposé afin de focaliser l'énergie électrique et thermique sur le bord exposé (130b, 1130b) défini le long d'une longueur de la lame de plasma.

2. Ensemble effecteur terminal selon la revendication 1, la plaque de scellement tissulaire électriquement conductrice du second élément de mâchoire ayant une configuration en forme de T ouvert définissant un canal le long d'une longueur de celle-ci et un élément isolant (115, 1115) étant disposé à l'intérieur du canal de la plaque de scellement tissulaire électriquement conductrice du second élément de mâchoire en alignement vertical opposé à la lame de plasma.

3. Ensemble effecteur terminal selon la revendication 2, l'élément isolant (115, 1115) étant réalisé à partir d'un silicone souple haute température.

4. Ensemble effecteur terminal selon la revendication 2 ou la revendication 3, l'élément isolant (115, 1115) étant choisi dans le groupe constitué par la céramique, le parylène, le nylon et le PTFE.

5. Ensemble effecteur terminal selon l'une quelconque des revendications précédentes, comprenant en outre un pont (122b) disposé à l'intérieur du premier élément de mâchoire à une extrémité proximale de celui-ci, le pont étant configuré pour fournir une continuité électrique à travers les plaques de scellement tissulaire électriquement conductrices des premier et second éléments de mâchoire.

6. Ensemble effecteur terminal selon l'une quelconque des revendications précédentes, comprenant en outre un capteur (700) associé de manière opérationnelle à au moins un des éléments de mâchoire et configuré pour détecter lorsque les éléments de mâchoire sont disposés dans la configuration ouverte, le capteur communiquant avec la source d'énergie électrique pour configurer l'instrument électrochirurgical pour une utilisation monopolaire lors de son activation.

7. Ensemble effecteur terminal selon l'une quelconque des revendications précédentes, comprenant en outre un commutateur d'activation bipolaire (80) configuré pour fournir de l'énergie électrique aux deux plaques de scellement tissulaire électriquement conductrices lors de leur activation et un commutateur d'activation monopolaire (90) configuré pour fournir de l'énergie électrique à la lame de plasma (130, 1130) lors de son activation.

8. Ensemble effecteur terminal selon la revendication 6, comprenant en outre un commutateur d'activation bipolaire (80) configuré pour fournir de l'énergie électrique aux deux plaques de scellement tissulaire électriquement conductrices lors de leur activation et un commutateur d'activation monopolaire (90) configuré pour fournir de l'énergie électrique à la lame de plasma lors de leur activation, le capteur (700) désactivant l'alimentation du commutateur d'activation bipolaire lorsque les éléments de mâchoire sont disposés dans la configuration ouverte.
